## Europäisches Patentamt
## European Patent Office
## Office européen des brevets

(11) Publication number: **0 082 481**
**B2**

# (12) NEW EUROPEAN PATENT SPECIFICATION

(45) Date of publication of the new patent specification: **12.09.90**

(51) Int. Cl.⁵: **A 61 K 37/66**

(21) Application number: **82111665.4**

(22) Date of filing: **15.12.82**

(54) Stabilised alpha-interferon formulations and their preparation.

(30) Priority: **23.12.81 US 334052**
**19.11.82 ZA 828580**

(43) Date of publication of application:
**29.06.83 Bulletin 83/26**

(45) Publication of the grant of the patent:
**13.03.85 Bulletin 85/11**

(45) Mention of the opposition decision:
**12.09.90 Bulletin 90/37**

(84) Designated Contracting States:
**AT BE CH DE FR GB IT LI LU NL SE**

(56) References cited:
**EP-A-0 027 573**    **GB-A-1 395 090**
**DE-A-2 629 808**    **GB-A-2 064 545**
**DE-A-2 917 899**    **US-A-3 940 480**
**FR-A-2 505 657**

(73) Proprietor: **SCHERING CORPORATION**
**2000 Galloping Hill Road**
**Kenilworth New Jersey 07033 (US)**

(72) Inventor: **Kwan, Henry King Hong**
**37 Knob Hill Drive**
**Summit New Jersey 07901 (US)**

(74) Representative: **Ritter, Stephen David et al**
**Mathys & Squire 10 Fleet Street**
**London EC4Y 1AY (GB)**

(56) References cited:
BIOLOGICAL ABSTRACTS, vol. 68, no. 8, 1979, no. 49099, Philadelphia, Pa., (USA); A.S.Levine
Menge et al "Enzyme Microb. Technol.6,101-112,1984
Cantell et al "In Vitro Monograph 3,35-38, 1974
Alfons Billiau et al., Antimicrobial Agents and Chemotherapy, Vol.16, pp. 49-55,1979
Contr.Inst. of Low Temp. Sci., B17, 1-20 (1972) ibid, 21-38 (1972)
The Interferon System, pp.135-157 (1979)
Proc.Natl.Acad.Sci.USA, 77,5928-5932 (1980)
J. Food Sci., 31, 293-299 (1966)

EP 0 082 481 B2

## Description

The present invention relates to α-interferon formulations having improved stability and to methods for their preparation.

The formulations of the present invention are useful in preparing sterile solutions, especially for injection or for use as nasal sprays, nasal solutions or ophthalmic solutions, or in the preparation of ointments, wherein α-interferon is the active drug. α-Interferon shows great potential in the treatment of a wide variety of disease states and particularly of various types of viral infections.

The well-known instability of α-interferon solutions makes it difficult to formulate stable compositions for clinical or veterinary use. Accordingly, it has been proposed to package α-interferon in lyophilised form for reconstitution with sterile water. Such a composition will contain a buffer to maintain a pharmaceutically acceptable pH when the solution is reconstituted and also sufficient sodium chloride to make the reconstituted solution isotonic. However, even in such a lyophilised composition, the α-interferon shows very limited and insufficient stability.

We have surprisingly discovered that the incorporation of certain amino acids and simple derivatives thereof into lyophilised α-interferon compositions yields a pharmaceutically acceptable lyophilised product that has significantly improved stability. Furthermore, it is possible by this means to obtain further advantages such as greater ease in the lyophilisation step itself, better reconstitution of the lyophilised product and better appearance of the product (in particular less colouration).

The present invention therefore provides a lyophilised pharmaceutical composition, for reconstitution with sterile water, comprising α-interferon together with a stabilising amount of an amino acid or derivative thereof selected from glycine, α-alanine and pharmaceutically acceptable salts thereof, and a compatible buffer system designed to maintain a pH of about 6.5 to 8.0 after reconstitution. Conveniently, there are about $1 \times 10^4$ to $5 \times 10^8$, preferably $1 \times 10^6$ to $1 \times 10^8$, International Units (I.U.) of α-interferon present for every 5 to 150 mg of amino acid or derivative thereof, the weight of any derivative of the amino acid being calculated as free amino acid.

The α-interferon used in the compositions according to the invention is preferably a human α-interferon or another α-interferon suitable for use in humans, or a polypeptide exhibiting biological properties similar to those of such an α-interferon. In particular, the human α-interferon may be a leukocyte interferon, and may have been prepared either by cultivation of leukocytes or by cultivation of microorganisms suitably programmed, through genetic engineering, i.e. by recombinant DNA methods, to produce such α-interferons or equivalent polypeptides.

It should be noted that a number of α-interferon species are known, usually designated by a numeral after the Greek letter. Also included within the scope of this invention are the so-called hybrid interferons wherein fragments of two or more native interferon species are joined. See, for example, EPA 51873 wherein hybrids of α-interferons are described. An example of such a hybrid α-interferon comprises the first 92 amino acids of α-1 interferon joined to a fragment comprising amino acids 92—165 of α-2 interferon (carboxy terminal portion). A particularly preferred form of α-interferon for use in the formulations of the present invention is α-2 interferon, especially α-2 interferon prepared by recombinant DNA methods, for example those disclosed by Nagata et al, in "Nature", Vol. 284, pages 316—320 (1980). Another preferred form of α-interferon for use in the formulations of the present invention is α-1 interferon, especially α-1 interferon prepared by recombinant DNA methods.

The specific activity of the α-2 interferon used in the formulations of the present invention should desirably be at least $5 \times 10^7$ I.U./mg. protein (of the interferon component), and preferably at least $1 \times 10^8$ I.U./mg. protein. Specific activity may be determined by measuring the antiviral activity as compared to the NIH reference standard and by measuring the total protein content using standard methods (e.g. the Lowry method). Similarly, high specific activity is desirable for clinical use of other interferons. At high levels of specific activity maintaining stability has hitherto been particularly difficult.

The amino acids are preferably used as the amino acids themselves, that is a glycine and α-alanine. Under these circumstances, about 5 to 150 mgs., preferably 5 to 25 mg, especially 7 to 22 mg, of α-alanine or especially of glycine will be present for $1 \times 10^4$ to $5 \times 10^8$ I.U. of interferon.

A composition containing these amounts of the ingredients is suitable for reconstitution by 1 ml. of sterile water.

The buffer system present in the compositions according to the invention is selected to be physiologically compatible and to maintain a desired pH in the reconstituted solution and also in the solution before lyophilisation. The pH of the reconstituted compositions of the present invention, especially those comprising α-2 interferon, should be about 6.5 to 8.0, preferably about 7.0 to 7.4. A preferred buffer system especially for compositions of α-2 interferon comprises disodium hydrogen phosphate and monosodium dihydrogen phosphate.

Whether an amino acid itself or a salt thereof is used, the buffer will be chosen to adjust the pH of the composition (after reconsitution) to a value within the desired range as indicated above.

Further components may be present in the compositions according to the invention, provided that they are physiologically compatible and are in no way detrimental to the interferon. In particular, a further stabiliser may be added. A preferred further stabiliser is albumin, especially human albumin when the compositions are designed for clinical use. For the above-mentioned quantity of amino acid (or derivative

2

thereof calculated as amino acid), namely 5 to 150 mg, (in particular preferably 5 to 25 mgs. of glycine), up to about 10 mg. of albumin, especially about 1 mg, may be added.

If the amount of α-interferon in a mililiter of reconstituted solution is to be less than about $5 \times 10^6$ I.U., the addition of albumin is very desirable.

A particularly preferred embodiment of the present invention contains the cited ingredients in the following proportions.

$1 \times 10^6$ to $1 \times 10^8$ of α-interferon (especially α-2 interferon);

about 5 to 25 mg, preferably 7 to 22 mg, of α-alanine or especially of glycine;

a compatible buffer system designed to provide a pH of about 7.0 to 7.4 in the reconstituted solution;

and about 1 miligram of albumin.

The invention further provides a process for the preparation of a novel composition as defined above which comprises the steps of: dissolving the amino acid or derivative thereof selected from glycine, α-alanine and pharmaceutically acceptable salts thereof, a compatible buffer system, optionally human albumin, and the interferon in sterile water; and lyophilising the resulting solution.

The water used in reconstituting the compositions of the present invention may also contain compatible ingredients, in particular preservatives such as methyl or isopropyl p-hydroxy-benzoate. Such preservatives are advantageous where the resulting solutions will be used not as a single dose but for multiple applications, for example as nasal or ophthalmic solutions.

The following non-limiting examples illustrate the preparation of a sterile lyophilised powder for preparing injectable solutions of an interferon; the preferred interferon is α-2 interferon.

### Example 1
Solution for Lyophilisation (1000 vials; 1 ml. per vial)

| Formula | |
|---|---|
| Interferon | $7.5 \times 10^{10}$ I.U. |
| Disodium Hydrogen Phosphate, Anhydrous, USP, Reagent | 2,27 grams per liter. |
| Sodium Dihydrogen Phosphate, USP | 0.55 gram per liter. |
| Glycine, USP | 20.0 grams per liter. |
| Human Albumin, USP | 1.0 gram per liter. |
| Water for Injection, USP q.s. ad | 1.0 liter. |

Method of manufacture:
1. Charge a portion of the water into a suitable vessel equipped with an agitator.
2. Charge and dissolve with agitation the sodium phosphates.
3. Charge and dissolve with agitation the glycine.
4. Charge and dissolve with agitation the albumin.
5. Charge and dissolve with agitation the Interferon.
6. Bring the batch to final volume with Water for Injection, USP.
7. In a sterile area, aseptically filter the solution into a sterilized vessel through a sterilised 0.2 micron filter which has been washed and tested for integrity. Test the integrity of the filter after filtration.
8. Aseptically fill the solution into sterilised vials.
9. Aseptically load the filled vials into a sterilised lyophiliser.
10. Aseptically lyophilise the solution.
11. Aseptically stopper the vials.
12. Apply the seal and crimp the vials.

Method of lyophilisation (using a shelf lyophiliser)
1. Precool the shelves to −30°C.
2. Load the vials into the chamber.
3. Allow the solutions to freeze. Cool the condenser to −45°C or below. Start the vacuum. Maintain shelf temperature at −30°C for at least 12 hours.
4. Increase shelf temperatures at a relatively constant rate to no more than +25°C in a period of no less than about 24 hours.
5. When the product temperature reaches +25°C, flood the chamber with sterile nitrogen until it reaches atmospheric pressure. Stopper the vials in the chambers.
6. Remove the vials from the chamber and seal them.

## Example 2

A preferred formulation of the present invention wherein the interferon is α-2 interferon is prepared by following the procedure of Example 1, using α-2 interferon as the interferon but substituting α-interferon for glycine.

## Example 3

A preferred formulation of the present invention wherein the interferon is α-1 interferon is prepared by following the procedure of Example 1 but using α-1 interferon as the interferon.

## Example 4

Another preferred formulation of the present invention is prepared by following the procedure of Example 1 but using α-1 interferon as the interferon and substituting α-alanine for glycine.

Per cent recovery of α-2 Interferon from different formulations under different storage conditions.

| Storage time (months) | Storage temperature (°C) | *Formulations I | II | III |
|---|---|---|---|---|
| Initial | | 100 | 100 | 100 |
| | | % recovery of α-2 Interferon | | |
| 1 | 25 | — | — | 83 |
| 3 | 4 | 89 | 67 | — |
| 3 | 25 | 83 | — | 23 |
| 6 | 4 | 106 | 52 | — |
| 6 | 25 | 106 | — | — |
| 6 | 35 | 115 | — | — |

*Formulations:
I: According to present invention, lyophilised;
II: Phosphate buffer with saline, pH 7,4, solution
III: Formulation II, lyophilised.

## Claims

1. A lyophilised pharmaceutical composition for reconstitution with sterile water, comprising α-interferon, a stabilising amount of an amino acid or derivative thereof selected from glycine, α-alanine and pharmaceutically acceptable salts thereof, and a compatible buffer system designed to maintain a pH of about 6.5 to 8.0 after reconstitution.

2. A composition as claimed in claim 1 wherein there are about $1 \times 10^4$ to $5 \times 10^8$ International Units of α-interferon present for every 5 to 150 mg of amino acid or derivative thereof, the weight of any derivative of the amino acid being calculated as the free amino acid.

3. A composition as claimed in claim 1 or claim 2 comprising also up to about 10 mg, preferably about 1 mg, of human albumin for every 5 to 150 mg of amino acid or pharmaceutically acceptable salt thereof.

4. A composition as claimed in any of the claims 1 to 3 wherein the buffer system is designed to maintain a pH of about 7.0 to 7.4 after reconstitution.

5. A composition as claimed in any of the claims 1 to 4 wherein the buffer system comprises disodium hydrogen phosphate and monosodium dihydrogen phosphate.

6. A composition as claimed in any of the claims 1 to 5 wherein the α-interferon is a human interferon, preferably α-2 interferon or α-1 interferon.

7. A composition as claimed in any of claims 1 to 6 wherein the α-interferon has been prepared by recombinant DNA methods.

8. A composition as claimed in any of claims 1 to 7 wherein there are by 5 to 25 mg, preferably 7 to 22 mg, of glycine for every $1 \times 10^4$ to $5 \times 10^8$ International Units of α-interferon.

9. A composition as claimed in any of claims 1 to 7 containing $1 \times 10^6$ to $1 \times 10^8$ International Units of α-interferon for every 5 to 150 mg, preferably 5 to 25 mg, especially 7 to 22 mg, of α-interferon, glycine or pharmaceutically acceptable salts thereof, the weight of any derivative of the glycine or α-interferon being calculated as free amino acid.

10. A composition as claimed in any of claims 1 to 9 comprising: $1 \times 10^6$ to $1 \times 10^8$ International Units of α-interferon, especially α-2 interferon;

about 5 to 25 mg, preferably 7 to 22 mg, of α-alanine or especially glycine;
a compatible buffer system, especially a mixture of disodium hydrogen phosphate and monosodium dihydrogen phosphate, designed to provide a pH of bout 7.0 to 7.4 in reconstituted solution;
and about 1 mg of human albumin.

11. A process for the preparation of a composition as claimed in claim 1 which comprises the steps of: dissolving the amino acid or a derivative thereof selected from glycine, α-alanine and pharmaceutically acceptable salts thereof, a compatible buffer system designed to provide a pH of about 6.5 to 8.0 in the reconstituted solution, optionally human albumin, and the α-interferon, in sterile water; and lyophilising the resulting solution.

12. A method for stabilising of a lyophilised pharmaceutical composition for reconstitution with sterile water to a pH of about 6.5 to 8.0 after reconstitution, comprising α-interferon and a compatible, suitable buffer system, which comprises incorporating a stabilising amount of an amino acid or derivative thereof selected from glycine, α-alanine and pharmaceutically acceptable salts thereof into the composition before lyophilisation.

## Patentansprüche

1. Lyophilisierte pharmazeutische Zusammensetzung zur Rekonstitution mit sterilem Wasser, enthaltend α-Interferon, eine stabilisierende Menge einer Aminosäure oder eines Derivats derselben ausgewählt aus Glycin, α-Alanin und deren pharmazeutisch unbedenklichen Salzen sowie ein verträgliches Puffersystem, um einen pH von etwa 6,5 bis 8,0 nach Rekonstitution aufrecht zu erhalten.

2. Zusammensetzung nach Anspruch 1, dadurch gekennzeichnet, daß etwa $1 \times 10^4$ bis $5 \times 10^8$ Internationale Einheiten α-Interferon auf jeweils 5 bis 150 mg der Aminosäure oder ihres Derivats vorliegen, wobei das Gewicht irgendeines Derivats der Aminosäure als freie Aminosäure berechnet wird.

3. Zusammensetzung nach Anspruch 1 oder Anspruch 2, dadurch gekennzeichnet, daß sie außerdem bis zu etwa 10 mg, vorzugsweise etwa 1 mg, Humanalbumin auf jeweils 5 bis 150 mg der Aminosäure oder ihres pharmazeutisch unbedenklichen Salzes enthält.

4. Zusammensetzung nach irgendeinem der Ansprüche 1 bis 3, dadurch gekennzeichnet, daß das Puffer-System so eingerichtet ist, daß es einen pH von etwa 7,0 bis 7,4, nach der Rekonstitution aufrechterhählt.

5. Zusammensetzung nach irgendeinem der Ansprüche 1 bis 4, dadurch gekennzeichnet, daß das Puffer-System Dinatriumhydrogenphosphat und Mononatriumhydrogenphosphat enthält.

6. Zusammensetzung nach irgendeinem der Ansprüche 1 bis 5, dadurch gekennzeichnet, daß das α-Interferon ein Human-Interferon vorzugsweise α-2-Interferon oder α-1-Interferon, ist.

7. Zusammensetzung nach irgendeinem der Ansprüche 1 bis 6, dadurch gekennzeichnet, daß das α-Interferon durch DNA-Rekombinationsmethoden hergestellt ist.

8. Zusammensetzung nach irgendeinem der Ansprüche 1 bis 7, dadurch gekennzeichnet, daß 5 bis 25 mg, vorzugsweise 7 bis 22 mg, Glycin auf jeweils $1 \times 10^4$ bis $5 \times 10^8$ Internationale Einheiten α-Interferon vorliegen.

9. Zusammensetzung nach irgendeinem der Ansprüche 1 bis 7, dadurch gekennzeichnet, daß sie $1 \times 10^6$ bis $1 \times 10^8$ Internationale Einheiten α-Interferon auf jeweils 5 bis 150 mg, vorzugsweise 5 bis 25 mg, insbesondere 7 bis 22 mg, α-Alanin, Glycin oder deren pharmazeutisch unbedenklicher Salze enthält, wobei das Gewicht irgendeines Derivats des Glycins oder α-Alanins als freie Aminosäure berechnet wird.

10. Zusammensetzung nach irgendeinem der Ansprüche 1 bis 9, dadurch gekennzeichnet, daß sie $1 \times 10^6$ bis $1 \times 10^8$ Internationale Einheiten α-Interferon, insbesondere α-2-Interferon, etwa 5 bis 25 mg, vorzugsweise 7 bis 22 mg, α-Alanin oder insbesondere Glycin, ein verträgliches Puffer-System, insbesondere eine Mischung aus Dinatriumhydrogenphosphat und Mononatriumhydrogenphosphat, zur Herstellung eines pH von etwa 7 bis 7,4 in der rekonstituierten Lösung sowie etwa 1 mg Human-Albumin enthält.

11. Verfahren zur Herstellung einer Zusammensetzung nach Anspruch 1 mit den Schritten: Auflösen der Aminosäure oder eines Derivats derselben ausgewählt aus Glycin, α-Alanin und deren pharmazeutisch unbedenklichen Salzen, eines verträglichen Puffer-Systems zur Herstellung eines pH von etwa 6,5 bis 8,0 in der rekonstituierten Lösung, gegebenenfalls des Human-Albumins, und des α-Interferons in sterilem Wasser und Lyophilisieren der erhaltenen Lösung.

12. Verfahren zur Stabilisierung einer lyophilisierten pharmazeutischen Zusammensetzung zur Rekonstitution mit sterilem Wasser auf einen pH von etwa 6,5 bis 8,0 nach Rekonstitution, enthaltend α-Interferon und ein verträgliches geeignetes Puffer-System, dadurch gekennzeichnet, daß eine stabilisierende Menge einer Aminosäure oder eines Derivats derselben ausgewählt aus Glycin, α-Alanin und deren pharmazeutisch unbedenklichen Salzen vor dem Lyophilisieren in die Zusammensetzung eingearbeitet wird.

## Revendications

1. Composition pharmaceutique lyophilisée pour reconstitution avec de l'eau stérile, comprenant l'α-interféron, un quantité stabilisante d'un acide aminé ou sel de celui-ci choisi parmi glycine, α-alanine et

leurs sels acceptables en pharmacie, et une systéme tampon compatible, conçu pour maintenir un pH d'environ 6,5 à 8,0 après reconstitution.

2. Composition selon la revendication 1 caractérisée en ce qu'elle comprend environ $1 \times 10^4$ à $5 \times 10^8$ Unités Internationales d'$\alpha$-interféron pour chaque 5 à 150 mg d'acide aminé ou dérivé de celui-ci, le poids de tout dérivé d'acide aminé étant calculé par rapport à l'acide aminé libre.

3. Composition selon la revendication 1 ou 2 caractérisée en ce qu'elle comprend également jusqu'à environ 10 mg, et de préférence 1 mg d'albumine humaine pour chaque 5 à 150 mg d'acide aminé ou sel acceptable en pharmacie de celui-ci.

4. Composition selon l'une des revendications 1 à 3, caractérisée en ce que le système tampon est destiné à maintenir un pH d'environ 7,0 à 7,4 après reconstitution.

5. Composition selon l'une des revendications 1 à 4, caractérisée en ce que le système tampon comprend de l'hydrogéno phosphate disodique et du dihydrogéno phosphate monosodique.

6. Composition selon l'une des revendications 1 à 5, caractérisée en ce que l'$\alpha$-interféron est un interféron humain, de préférence $\alpha$-2 interféron ou $\alpha$-1 interféron.

7. Composition selon l'une des revendications 1 à 6, caractérisée en ce que l'$\alpha$-interféron a été préparé par des procédés de recombinaison d'ADN.

8. Composition selon l'une des revendications 1 à 7, caractérisée en ce qu'elle comprend 5 à 25 mg, de préférence 7 à 22 mg, de glycine pour chaque $1 \times 10^4$ à $5 \times 10^8$ Unités Internationales d'$\alpha$-interféron.

9. Composition selon l'une des revendications 1 à 7, caractérisée en ce qu'elle comprend $1 \times 10^6$ à $1 \times 10^8$ Unités Internationales d'$\alpha$-interféron pour chaque 5 à 150 mg , de préférence 5 à 25 mg, et spécialement 7 à 22 mg d'$\alpha$-alanine, glycine ou de leurs sels acceptables en pharmacie, le poids de tout dérivé de glycine ou $\alpha$-alanine étant calculé par rapport à l'acide aminé libre.

10. Composition selon l'une des revendications 1 à 9, caractérisée en ce qu'elle comprend $1 \times 10^6$ à $1 \times 10^8$ Unités Internationales d'$\alpha$-interféron, et spécialement $\alpha$-2 interféron; environ 5 à 25 mg, et de préférence 7 à 22 mg d'$\alpha$-alanine ou en particulier de glycine; un système tampon compatible, en particulier un mélange d'hydrogéno phosphate disodique et de dihydrogéno phosphate monosidique, destiné à fournir un pH d'environ 7,0 à 7,4 dans la solution reconstituée, et environ 1 mg d'albumine humaine.

11. Procédé de préparation d'une composition selon la revendication 1 caractérisé en ce qu'il comprend les étapes de: dissoudre l'acide aminé ou dérivé de celui-ci choisi parmi glycine, $\alpha$-alanine et sels acceptables en pharmacie de ceux-ci, un système tampon compatible, conçu pour produire un pH d'environ 6,5 à 8,0 dans la solution reconstituée, éventuellement de l'albumine humaine, et l'$\alpha$-interféron, dans de l'eau stérile; et lyophiliser la solution résultante.

12. Procédé pour la stabilisation d'une composition pharmaceutique lyophilisée pour reconstitution avec de l'eau stérile à une pH d'environ 6,5 à 8,0 après reconstitution, comprenant l'$\alpha$-interféron et un système tampon compatible et approprié, caractérisé en ce qu'il comprend l'incorporation d'une quantité stabilisante d'un acide aminé ou dérivé de celui-ci choisi parmi glycine, $\alpha$-alanine et sels acceptables en pharmacie de ceux-ci dans la composition avant lyophilisation.